(19) Europäisches Patentamt

European Patent Office

Office européen des brevets

(11) **EP 3 592 433 B1**

(12) **FASCICULE DE BREVET EUROPEEN**

(45) Date de publication et mention de la délivrance du brevet:
**03.11.2021 Bulletin 2021/44**

(21) Numéro de dépôt: **18712956.4**

(22) Date de dépôt: **06.03.2018**

(51) Int Cl.:
*A61Q 5/12* *(2006.01)*     *A61K 8/73* *(2006.01)*
*A61Q 5/02* *(2006.01)*     *A61K 8/9783* *(2017.01)*

(86) Numéro de dépôt international:
**PCT/FR2018/050505**

(87) Numéro de publication internationale:
**WO 2018/162833 (13.09.2018 Gazette 2018/37)**

(54) **UTILISATION D'EXTRAITS D'AGAVE POUR PROTÉGER LA FIBRE CAPILLAIRE**

VERWENDUNG VON AGAVENEXTRAKTEN ZUM SCHUTZ DER HAARE

USE OF AGAVE EXTRACTS TO PROTECT KERATIN FIBERS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.03.2017 FR 1751833**

(43) Date de publication de la demande:
**15.01.2020 Bulletin 2020/03**

(73) Titulaire: **Laboratoires De Biologie Vegetale Yves Rocher**
**56200 La Gacilly (FR)**

(72) Inventeurs:
• **TUFEU, Sylvie**
  **75020 Paris (FR)**
• **ONGENAED, Julie**
  **92130 Issy-les-Moulineaux (FR)**
• **TCHALEKIAN, Sevag**
  **92130 Issy-les-Moulineaux (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**EP-A1- 1 541 117     EP-A1- 1 967 178**

## Description

### Domaine technique

La présente invention se rapporte à l'utilisation cosmétique non thérapeutique de fructanes pour protéger la fibre capillaire par amélioration de l'hydrophobicité de la fibre capillaire.

La présente invention trouve son application dans le domaine de la cosmétique, et plus particulièrement de la cosmétique capillaire.

Dans la description ci-dessous, les références entre crochets ([ ]) renvoient à la liste des références présentée à la fin du texte.

### Etat de la technique

A l'instar de la peau, les cheveux accumulent les dégâts engendrés par les stress environnementaux comme les UVs, la pollution, le froid, mais également par les traitements esthétiques comme le séchage, le frisage, le lissage, la décoloration et la coloration, ou l'utilisation de produits agressifs lors de shampoing trop récurrents ou mal adaptés. De plus, ils sont affectés par des facteurs internes comme le stress, les modifications hormonales ou encore le type d'alimentation.

Le cheveu est constitué, dans sa partie visible, de 3 couches :

- la cuticule, d'une épaisseur de 3,5 à 4,5 $\mu$m, est la couche la plus externe. Elle est constituée de plaques cornées en forme d'écailles qui se recouvrent partiellement et constitue une protection imperméable pour la tige du cheveu,
- vient ensuite le cortex, représentant 80% du cheveu. Il est composé principalement de protéines (kératine essentiellement) et confère à la fibre sa résistance et solidité de par sa haute organisation. Le cortex contient les granules de mélanines, donnant au cheveu sa couleur,
- enfin la moelle, composée de cellules mortes, qui a une texture molle et graisseuse.

La couche externe de la cuticule et donc de la surface des cheveux est constituée d'acides gras. Ces acides gras liés et non liés présents à la surface des cheveux agissent comme une couche protectrice rendant la surface des cheveux hydrophobe. L'hydrophobicité des cheveux est un paramètre important pour les conserver en bonne santé. En principe, cette propriété permet d'éviter une trop longue présence d'eau favorable au développement des bactéries. De plus, cette barrière permet de limiter et/ou d'empêcher la pénétration d'autres composés présents dans l'environnement extérieur. Un cheveu en bon état peut tout de même absorber plus de 30% de son propre poids en eau. Sa longueur peut alors augmenter de 2% et son diamètre de 15% à 20%.

Les agressions à répétition détériorent ce film lipidique. Lorsque la cuticule est endommagée, le cheveu devient trop poreux, et le phénomène de pénétration de l'eau est amplifié, le cheveu pouvant alors absorber 45% de son propre poids en eau. Cette absorption massive d'eau déstabilise les interactions entre les protéines et lui fait donc perdre force et résistance.

Une cuticule endommagée correspond ainsi à un cheveu trop hydrophile venant donc d'une détérioration de sa cuticule qui ne joue plus son rôle de barrage naturel, devant le cortex. Plus la cuticule de la tige capillaire est altérée, voire détruite, plus la kératine du cortex absorbe l'eau et perd de sa tenue et de son élasticité.

Parmi les matières premières cosmétiques utilisées pour le soin des cheveux se trouvent les agents conditionneurs qui ont pour but de rendre le cheveu plus facile à coiffer, plus doux, plus brillant.

Ces agents conditionneurs peuvent être des tensioactifs cationiques ou des dérivés siliconés.

On trouve également des inulines modifiées par greffage chimique d'un groupement à caractère hydrophobe sur la structure moléculaire de base de l'inuline (WO200790554 **([1])).** Ce groupement chimique peut être de différentes formes et correspond le plus souvent à une chaine carbonée hydrophobe. Cette modification confère les propriétés de conditionneur à ces inulines. Ces inulines proviennent la plupart du temps de la source végétale la plus classiquement utilisée pour leur extraction : la chicorée .

Toutefois, il reste un réel besoin de trouver de nouvelles compositions et/ou composés d'origine végétale et/ou naturels permettant d'améliorer de façon effective les propriétés protectrices des cheveux endommagés, et en particulier les propriétés d'hydrophobicité.

### Description de l'invention

La présente invention a précisément pour but de répondre à ces besoins et inconvénients de l'art antérieur.

Les inventeurs sont les tout premiers à utiliser des extraits d'Agave permettant précisément de répondre efficacement aux besoins précités.

La Demanderesse a identifié, de manière surprenante, que certains fructanes, et notamment certaines inulines naturelles *d'Agave tequilana,* c'est-à-dire non modifiées chimiquement, possèdent une action hydrophobe permettant une protection de la fibre capillaire, et ce malgré leur caractère hydrophile.

La Demanderesse a en effet mis en évidence, au termes d'importantes recherches, que l'utilisation de certaines inulines permet d'améliorer significativement l'hydrophobicité de la fibre capillaire, notamment des cheveux endommagés.

De manière surprenante, les inulines utilisées dans le cadre de l'invention, en dépit de leur caractère hydrophile, permettent une amélioration des propriétés hydrophobes du cheveu.Ainsi, un premier objet de l'invention se rapporte à l'utilisation cosmétique non thérapeutique de fructanes *d'Agave tequilana* comprenant des inulines naturelles pour protéger la fibre capillaire par amélioration de l'hydrophobicité de la fibre capillaire

L'agave est une plante monocotylédone, actuellement classée dans la famille des Agavaceae. Les espèces les plus courantes sont *Agave tequilana, Agave americana* et *Agave attenuata.* L'agave est endémique sur le continent américain et est présente du sud du Canada jusqu'au Nord de l'Amérique du Sud et les îles Caraïbes, en particulier au Mexique et dans toute l'Amérique centrale (Maria de la Soledad ALONSO GUTIÉRREZ, thèse, « Valorisation de la bagasse de *l'Agave tequilana W. cv azul* : caractérisation, étude de la digestibilité et de la fermentation des sucres », 2005 **([2])).**

Les agaves présentent une partie souterraine ayant la forme d'un rhizome, et une partie aérienne présentant de longues tiges et de grandes feuilles fibreuses, disposées en forme de rosette et qui finissent en épine. Les fleurs se trouvent à la fin de la tige et sont de couleur jaune-vert (ALONSO GUTIÉRREZ **([2])).** L'inuline est un polysaccharide de réserve trouvé dans les plantes de la famille des Artéracées, notamment dans les bulbes et racines de dahlias, de topinambour, d'artichauts, de pissenlits et dans la chicorée, cette dernière étant la source la plus utilisée industriellement. Constituée de 30 à 40 unités de fructose liées par des liaisons glycosidiques en $\beta$ (1-2), et d'une à deux molécules de glucose placées en bout de chaîne, l'inuline est en réalité un glucofructosane. Les inulines d'agaves sont des réserves glucidiques de type fructanes (également appelées fructosanes). Des polyfructosanes ont été extraits de *l'Agave tequilana Weber var. azul,* âgée de 8 ans. La caractérisation de ces fructosanes a donné comme résultat un degré de polymérisation compris entre 3 et 29, et une composition complexe de fructo-oligosacharides avec des liaisons de $\beta$ (1-2) et $\beta$ (2- 6) différentes des inulines rencontrées dans les autres plantes (López, M.G. ; Mancilla, N.A. ; Mendoza-Díaz, G. (2003). : Molecular structures of fructans from Agave tequilana Weber var. azul. J. Agric. Chem. 51, 7835-7840 **([3])** ; ALONSO GUTIÉRREZ **([2])).** Les inuline de chicorée ont une structure différente de celle des inulines d'agave. Les inulines de chicorée présentent en effet des chaines linéaires de fructoses liées par des liaisons beta 2-1 alors que les inulines d'agave, même si elles sont composées également principalement de fructose, ont des structres latérales branchées à la structure principale par des liaisons béta 2-6 (López, M.G. et al. **([3])).**

On entend par « fructanes *d'Agave tequilana* », au sens de la présente invention, des fructanes issus d'un extrait de la plante. L'extraction des fructanes peut être réalisée par tout procédé connu de l'homme du métier, par exemple par extraction des carbohydrates à partir de la bagasse, clarification de la liqueur brute obtenue à l'issue de l'extraction puis séparation pour obtenir une fraction de fructanes. Les fructanes *d'Agave tequilana* ont généralement un degré de polymérisation allant de 3 à 60, plus particulièrement de 3 à 29.

Plus particulièrement, l'utilisation de l'invention s'entend d'inulines naturelles.

On entend par « inulines naturelles », au sens de la présente invention, des fructanes *d'Agave tequilana* non modifiés chimiquement, notamment ne comportant pas de groupement à caractère hydrophobe greffé chimiquement. Ces inulines naturelles ont donc un caractère hydrophile, ce qui les différencie à double titre des inulines de chicorée modifiées chimiquement utilisées comme agents conditionneurs, à savoir par leur structure polysaccharidique et par leur comportement physico-chimique vis-à-vis de l'eau. Elles possèdent un degré de polymérisation supérieur à 10, pouvant par exemple aller de 10 à 60, ou de 10 à 29. Les inulines peuvent être obtenues par tout procédé connu de l'homme du métier, comme par exemple celui décrit par ALONSO GUTIÉRREZ **([2]).** Un exemple d'inuline naturelle peut être par exemple le produit Metlin® (NEKUTLI) caractérisé par des inulines vraies ayant un degré de polymérisation supérieur à 10.

On entend par « protéger la fibre capillaire », au sens de la présente invention, le fait de réparer, en tout ou partie, les dégâts engendrés par les stress environnementaux, par exemple les UVs, les traitements esthétiques comme le séchage, le frisage, le lissage et la coloration, ou l'utilisation de produits agressifs. Avantageusement, l'effet des fructanes, et notamment des inulines utilisées dans le cadre de l'invention, permet l'amélioration de l'hydrophobicité de la fibre capillaire.

On entend par « hydrophobicité de la fibre capillaire », au sens de la présente invention, le caractère hydrophobe de la surface du cheveu lié à la présence d'acides gras formant la couche externe de la cuticule.

De manière surprenante, l'effet d'amélioration de l'hydrophobicité de la fibre capillaire n'est pas lié aux propriétés hydrophobes de l'inuline elle-même, puisque les inulines utilisées dans l'invention ont un caractère hydrophile. Ainsi, les fructanes et notamment les inulines décrites dans l'invention permettent de nourrir le cheveu.

Avantageusement, l'amélioration de l'hydrophobicité est d'au moins 6%, par exemple entre 6 et 14% selon les ratio, par rapport à l'hydrophobicité mesurée avant utilisation des inulines selon l'invention. Cette amélioration peut être mesurée grâce à un test tensiometrique, comme par exemple le test de Wilhelmy sur fibre capillaire (SCHULZE ZUR WIESCHE et al. : « Prévention of hair surface aging », J. Cosmet. Sci., 62, 237-249 (March/April 2011) Henkel AG & Co. KGaA, Hohenzollemring 127-129, 22763 Hamburg, Germany (E.S.z.W.), DWI an der RWTH Aachen e.V., Pauwelsstr. 8, 52056 Aachen, Germany (A.K., K.S.), and School of Materials, The University of Manchester, Manchester M13 9PL, UK (F-J.W.) **([4])).**

Dans le cadre de l'utilisation de l'invention, les fructanes peuvent comprendre en outre des fructo-oligosaccharides ayant un degré de polymérisation inférieur ou égal à 10. Avantageusement, les fructo-oligosaccharides ayant un degré de polymérisation inférieur ou égal à 10 ont un effet d'amélioration de la croissance du cheveu et/ou de l'aspect du cheveu et/ou du cuir chevelu. Il peut s'agir par exemple d'un degré de polymérisation allant de 3 à 10, par exemple de 3 à 9, ou de 4 à 8. Il peut par exemple s'agir du produit commercial Metlos® (NEKUTLI), caractérisé par des fructo-oligosaccharides ayant un degré de polymérisation inférieur ou égal à 10.

Dans le cadre de l'invention, le ratio inulines sur fructo-oligosaccharides utilisé peut être compris de 100/0 à 0/100, la borne 0/100 n'étant éventuellement pas incluse. Le ratio peut être par exemple de 75/25, ou de 25/75, ou de 50/50.

Avantageusement, les fructanes sont présents dans une composition cosmétique comprenant un support cosmétiquement acceptable. Le support cosmétiquement acceptable peut être par exemple choisi dans le groupe comprenant les shampooings, les lotions, notamment les lotions capillaires, les crèmes, les mousses, les laits, les sprays, les suspensions et les sérums.

La composition cosmétique présente avantageusement tous les effets techniques conférés par l'extrait *d'Agave tequilana* décrit ci-avant, et peut comprendre en outre un véhicule acceptable et de 0,05 à 5 % en poids d'extraits d'Agave par rapport au poids total de la composition, ledit extrait d'agave comprenant des inulines ayant un degré de polymérisation compris de 10 à 60 et éventuellement des fructo-oligosaccharides ayant un degré de polymérisation inférieur ou égal à 10. Toutes les caractéristiques d'extraits d'Agave mentionnées ci-avant pour l'utilisation de l'invention, ainsi que toutes les caractéristiques de l'utilisation d'un extrait décrites ci-avant, s'appliquent mutatis mutandis à la composition de l'invention.

Avantageusement, la composition cosmétique peut comprendre un mélange de fructanes *d'Agave tequilana* constitué (i) d'inulines ayant un degré de polymérisation supérieur à 10, et (ii) de fructo-oligosaccharides ayant un degré de polymérisation inférieur ou égal à 10, dans laquelle le ratio d'inulines sur fructo-oligosaccharides est égal à 50/50.

On entend par « composition cosmétique », dans la présente invention, toute composition à visée cosmétique, c'est à dire esthétique, une composition pouvant être mise en contact avec les parties superficielles du corps humain, par exemple l'épiderme, les systèmes pileux et capillaires. Avantageusement, une composition cosmétique permet, exclusivement ou principalement de les protéger, parfumer, maintenir en bon état, modifier leur aspect ou en corriger les défauts esthétiques superficiels. La composition de l'invention peut notamment contenir un véhicule cosmétiquement acceptable.

Par « véhicule cosmétiquement acceptable », on entend un véhicule adapté pour une utilisation en contact avec des cellules humaines et animales cutanées, en particulier les cellules de l'épiderme, sans toxicité, irritation, réponse allergique indue et similaire, et proportionné à un rapport avantage/risque raisonnable.

Le véhicule cosmétiquement acceptable peut par exemple être choisi parmi l'eau, la glycérine, gel d'aloe vera, un extrait de plante aqueux, les tensio-actifs d'origine naturel, cette liste n'étant pas limitative.

La composition de l'invention peut être obtenue par tout procédé approprié connu de l'homme du métier pour la fabrication d'une composition cosmétique. Il peut s'agir, par exemple d'un simple mélange. Il peut s'agir alternativement, par exemple, d'un procédé comprenant une étape d'incorporation d'une phase interne dans une phase externe au moyen d'un émulseur, par exemple d'une turbine de type rotor-stator. Il peut s'agir également par exemple d'un procédé utilisant la Température d'Inversion de Phase (TIP), ce procédé étant classiquement utilisé par l'homme de l'art pour obtenir des émulsions huile dans eau dont les gouttelettes dispersées sont particulièrement fines, par exemple avec un diamètre de 0,1 à 1 $\mu$m.

La composition cosmétique peut comprendre de 0,05 à 5 % en poids d'extraits d'Agave par rapport au poids total de la composition, par exemple de 0,1 à 5%, de 0,1 à 1%, de 0,25 à 1% ou de 0,25 à 0,5%.

La composition cosmétique de la présente invention peut se trouver sous toute forme appropriée pour une application cosmétique. Avantageusement, la composition est une composition à usage topique, rincée ou non rincée.

Il peut s'agir par exemple d'une composition sous une forme choisie dans le groupe comprenant une émulsion huile dans eau ou eau dans huile ou un mélange de ces émulsions.

Selon l'invention, la composition cosmétique peut par exemple se trouver sous une forme choisie dans le groupe comprenant un gel aqueux ou hydroalcoolique, une crème aqueuse ou hydroalcoolique et une lotion aqueuse ou hydroalcoolique. Ces formulations utilisables pour la mise en œuvre de la présente invention sont connues dans l'état de la technique par les formulateurs. Dans ces exemples de composition, il suffit d'ajouter des extraits d'Agave de la présente invention pour obtenir une composition conforme à la présente invention.

Selon l'invention, la composition peut être sous une forme choisie parmi un shampooing, un onguent, une crème, une huile, un lait, une pommade, une poudre, une solution, un gel, un sérum, un baume, un beurre, une lotion, une suspension, un savon, un spray ou une émulsion, cette forme pouvant être rincée ou non rincée.

L'extrait de la présente invention peut être utilisé dans une composition cosmétique seul ou en combinaison avec d'autres substances ou ingrédients actifs ou inactifs cosmétiquement ou dermatologiquement. Les substances ou ingrédients inactifs sont ceux qui n'agissent pas cosmétiquement ou dermatologiquement. Il s'agit des éléments de la composition permettant notamment d'accompagner l'extrait, de constituer une formulation particulière, de conserver l'extrait actif dans le temps, cette liste n'étant pas limitative. Il peut s'agir en d'autres termes de tout produit de base pouvant se trouver dans les compositions cosmétiques ou dermatologiques classiques. Par opposition, les substances ou ingrédients actifs sont ceux qui dans l'application cosmétique ou dermatologique visée ont une action esthétique et/ou médicale.

Ainsi, l'extrait d'Agave de la présente invention peut être la seule substance ou ingrédient actif d'une composition, ou il peut être associé à d'autres substances ou ingrédients actifs d'une composition cosmétique ou dermatologique.

Un dispositif est également décrit dans le cadre de l'invention, pouvant se présenter sous une forme choisie parmi un pot, un flacon, un flacon-pompe, un tube, un sachet soudé, un masque, un spray, ledit dispositif comprenant un extrait ou une composition selon l'invention.

Un autre objet de l'invention se rapporte à un procédé cosmétique non thérapeutique comprenant l'application thérapeutique de fructanes *d'Agave tequilana* comprenant des inulines ayant un degré de polymérisation supérieur à 10, pour protéger la fibre capillaire, ladite application étant éventuellement suivie d'un rinçage.

Dans le cadre des procédés cosmétiques selon l'invention, ou de l'utilisation selon l'invention, l'utilisation s'entend d'une utilisation non-thérapeutique, par exemple pour le traitement des peaux et cheveux sains, c'est-à-dire notamment des cuirs chevelus ne présentant pas un état pathologique ou, si le cuir chevelu présente un état pathologique, pour une utilisation strictement esthétique, à l'exclusion de toute utilisation thérapeutique. En effet, l'utilisation cosmétique et esthétique de l'invention n'est pas associée à un effet thérapeutique inévitable.

Ainsi, toute utilisation cosmétique et tout procédé cosmétique selon l'invention sont respectivement des utilisations cosmétiques non-thérapeutiques et des procédés cosmétiques non-thérapeutiques, ne visant pas à traiter une pathologie.

Dans un mode de réalisation particulier, l'application n'est pas suivie d'un rinçage.

Quel que soit le mode de réalisation, l'application peut s'effectuer sur des cheveux naturels, des cheveux abîmés, des cheveux secs, des cheveux colorés ou des cheveux cassants.

D'autres avantages pourront encore apparaître à l'homme du métier à la lecture des exemples ci-dessous, illustrés par les figures annexées, donnés à titre illustratif.

## EXEMPLES

### Exemple 1 : Préparation des extraits d'Agave

Les extraits d'Agave peuvent être des produits commerciaux Metlos® (fructooligosaccharides de degré de polymérisation inférieur ou égal à 10), et/ou Metlin® (inulines de degré de polymérisation supérieur à 10), fournis par Nekutli.

La matière première utilisée pour la production de Metlos® et de Metlin® est l'agave bleue biologique récoltée dans les hauts plateaux de l'État de Jalisco.

Après 5 à 7 ans de croissance soigneusement contrôlée, l'agave a la quantité maximale de fructanes, et est donc prête à être transformée.

L'agave est reçue fraîche et lavée à l'arrivée pour éliminer autant de matières étrangères que possible. L'agave est ensuite broyée en trois étapes pour passer de 40 à 100 kg de noyau d'agave à une bagasse pulpeuse, prête à être transformée pour en extraire les glucides.

La liqueur brute obtenue à partir de l'extraction est ensuite clarifiée pour éliminer toute couleur et impuretés restantes. Ensuite, le jus clarifié est purifié en deux fractions: Metlos® et Metlin®. Chaque produit est ensuite amené à une dernière étape de purification afin d'éliminer tous les minéraux pour obtenir une pureté élevée (> 99% de glucides).

### Exemple 2 : Évaluation de l'effet des inulines par des mesures tensiométriques de la surface des cheveux

L'objectif de cette étude est d'évaluer l'effet de Metlin® et de Metlos®, notés respectivement actif A et actif B par des mesures de l'angle de contact de l'eau sur la surface des cheveux.

Les cheveux sains sont naturellement protégés par une couche de surface lipidique avec des propriétés hydrophobes.

Or, l'hydrophobicité des cheveux diminue après des dommages chimiques engendrés par exemple par la décoloration. Le but de la présente étude est de mesurer l'augmentation de l'hydrophobicité sur la surface des cheveux après immersion dans une solution d'actifs nourrissante à base de fructanes (inulines et/ou fructo-oligosaccharides), dues à l'amélioration de l'hydrophobicité sur les cheveux.

Principe de l'étude

La mesure est basée sur la méthode de Wilhelmy. Il s'agit d'une méthode actuellement utilisée pour mesurer l'angle de contact d'un liquide sur une seule fibre. La fibre (le cheveu) ne doit pas être perméable au liquide utilisé (l'eau) et la tension superficielle de ce liquide doit être connue.

Le principe consiste en l'utilisation d'une balance de haute précision afin de mesurer la force nécessaire lors de l'immersion des cheveux dans l'eau. L'angle de contact est calculé à partir de l'équation de Wilhelmy:

$$\gamma = \frac{F}{L \times \cos\theta}$$

Avec :

γ : Tension de surface de l'eau : 72,8 mN/m
F : force
L : longueur à l'état mouillé (mesuré précédemment)
θ : angle de contact

Comme indiqué ci-dessus, la mesure de l'angle de contact nécessite une détermination préalable de la longueur mouillée de l'échantillon. La mesure de la longueur mouillée s'effectue à l'aide d'un solvant à faible tension superficielle, l'hexane dont la tension superficielle est de 18,4 mN/m, pour laquelle on considère que l'angle de contact est nul.

Quant à la mesure de l'angle de contact, l'échantillon est graduellement immergé dans l'hexane, la force d'immersion est mesurée. La force F exercée juste en contact avec le solvant est déterminée par régression linéaire, la et par calcul, et la longueur mouillée avec l'équation de Wilhelmy.

Données du test

| Mèche | Naturelle - traitée |
|---|---|
| T Nat | Natural standard (bruns européens, fournisseur : Quimdis) |
| D2 | D2 - Standard |
| D2 + Trait ement | D2 +1 application par immersion rincée ou non dans la solution d'actif |

Origine des cheveux: Cheveux bruns européens.
Les cheveux dits « D2 » ou « D2 standard » sont des cheveux bruns européens (fournisseur : Quimdis) ayant subit 2 cycles de décolorations. La décoloration est l'éclaircissement de la nuance naturelle des cheveux par la modification chimique (procédés d'oxydation alcaline) des caractéristiques des pigments de mélanine. Le processus, très progressif, de décoloration du pigment (solubilisation par dépolymérisation de la mélanine) est précédé de l'attaque de la kératine et des polypeptides stabilisant le pigment mélanique par le traitement décolorant (rupture des liaisons disulfures, amides, salines et hydrogènes). On note D2 les cheveux ayant subi une décoloration répétée deux fois. Nous réalisons ce processus en laboratoire par immersion des mèches dans une solution à base de peroxodisulfate de sodium et de peroxyde d'hydrogène.

Les inulines ou oligo-fructosaccharides utilisées sont Metlin® ou Metlos® (Nekutli) et leurs mélanges dont les ratios Metlin®/ Metlos® sont les suivants : 100/0, 75/25, 50/50, 25/75 et 0/100.

Les concentrations des inulines et/ou oligo-fructosaccharides dans les solutions aqueuses d'actifs appliquées sont de 0,25%, 0,5% et 1%. Ce pourcentage correspond au produit total dans le produit, c'est-à-dire soit l'inuline lorsque le produit ne contient pas de oligo-fructosaccharides, soit les oligo-fructosaccharides lorsque le produit ne contient pas d'inuline, soit le mélange des deux lorsque le produit comporte de l'inuline et des oligo-fructosaccharides.

Dans certains cas, les cheveux ont été rincés après l'application des inulines et/ou oligo-fructosaccharides après 5 minutes d'immersion dans la solution aqueuse d'actifs, tandis que dans les autres cas, les cheveux n'ont pas été rincés.

Le tensiomètre utilisé est le tensiomètre Krüss K100SF.

Protocole d'échantillonnage

10 cheveux par mèche ont été mesurés.

Pour chaque cheveu, le protocole suivant est utilisé:

- Le cheveu est coupé de la mèche à l'aide de ciseaux propres sans autre manipulation et manipulés avec une pince propre.
- Il est fixé sur sa partie supérieure sur une feuille de papier au moyen d'un adhésif double face.
- Pour chaque mesure, un échantillon d'une longueur de 15 mm est coupé à l'aide de ciseaux propres.
- Les 3 premiers échantillons sont utilisés pour mesurer la longueur mouillée.
- Les 3 échantillons suivants sont utilisés pour mesurer l'angle de contact.
- L'échantillon est fixé sur la surface collante du porte-échantillon, sur une longueur d'environ 5 mm (fixation directe sur le porte-échantillon sans autre manipulation). L'échantillon doit être aussi droit que possible, pour venir perpendiculairement au contact du liquide, de manière à ne pas fausser la mesure.

La mesure est effectuée à 5 mm du fond de l'échantillon.

Conditions de mesure

Mesure de longueur mouillée

| paramètres: | valeur |
| --- | --- |
| Vitesse de détection | 6 mm/min |
| Sensibilité de détection | $5.10^{-5}$ g |
| Mesure de la vitesse | 3 mm/min |
| Différence de position | 0,2 mm |
| Profondeur d'immersion | 5 mm |

Contrôle de l'eau

Les mesures sont effectuées avec de l'eau du robinet qui est systématiquement contrôlée tous les jours lors des mesures.

Résultats:

| temperature | pH | Surface tension (mN/m) |
| --- | --- | --- |
| 23 °C | 7,4±1 | 72,7 mN/m (s=0.1) |

Résultats des tests d'hydrophobicité:

Les résultats sont donnés au moyen d'un calcul d'un test W de Mann-Whitney pour comparer les médianes des deux échantillons comparés, les échantillons étant indépendants. Les échantillons comparés sont les cheveux abimés par décoloration (D2) et les cheveux abimés par décoloration (D2) et traités avec la solution aqueuse d'actifs.

RESULTATS DES TESTS HYDROPHOBICITE 1% (Actif A : Inuline-Metlin®, Actif B : Metlos®)

| références | Mèche 1 : cheveux naturels Angle de contact (°) | | Mèche 2 : cheveux abîmés (décolorés) | | Angle de contact (°) | |
|---|---|---|---|---|---|---|
| | reg | | moy | | reg | moy |
| moy | 102,4 | | 101,5 | | 78,2 | 77,8 |
| Ecart type | 2,9 | | 2,4 | | 11,8 | 11,0 |
| Coef variation % | 2,8 | | 2,3 | | 15,1 | 14,1 |

| Ratio A/B | 100/0 | | 75/25 | | 50/50 | | 25/75 | | 0/100 | |
|---|---|---|---|---|---|---|---|---|---|---|
| Non rincé | Mèche 3 | | Mèche 4 | | Mèche 5 | | Mèche 6 | | Mèche 7 | |
| | reg | moy | reg | moy | reg | moy | reg | moy | reg | moy |
| moy | 91,8 | 90,2 | 83,7 | 83,7 | 87,1 | 85,2 | 84,5 | 82,6 | 84,4 | 82,7 |
| Ecart type | 7,4 | 7,8 | 13,5 | 12,3 | 13,2 | 13,6 | 11,5 | 11,5 | 15,2 | 15,7 |
| Coef variatio n % | 8,1 | 8,7 | 16,1 | 14,7 | 15,2 | 15,9 | 13,6 | 13,9 | 18,1 | 19,0 |

| Ratio A/B | 100/0 | | 75/25 | | 50/50 | | 25/75 | | 0/100 | |
|---|---|---|---|---|---|---|---|---|---|---|
| Rincé | Mèche 8 | | Mèche 9 | | Mèche 10 | | Mèche 11 | | Mèche 12 | |
| | reg | moy | reg | moy | reg | moy | reg | moy | reg | moy |
| moy | 90,8 | 90,5 | 87,7 | 85,7 | 87,7 | 84,7 | 83,4 | 82,1 | 87,4 | 85,9 |
| Ecart type | 11,6 | 11,4 | 7,0 | 7,4 | 10,6 | 11,4 | 9,5 | 8,8 | 8,3 | 8,7 |
| Coef variation % | 12,7 | 12,6 | 8,0 | 8,6 | 12,1 | 13,4 | 11,4 | 10,8 | 9,5 | 10,1 |

**Une différence significative par rapport au D2 est constatée.**

RESULTATS DES TESTS HYDROPHOBICITE 0.5% et 0.25% (Actif A : Inuline-Metlin®, Actif B : Metlos®)

| références | Mèche 1 : cheveux naturels Angle de contact (°) | | Mèche 2 : cheveux abîmés (décolorés) Angle de contact (°) | |
|---|---|---|---|---|
| | reg | moy | reg | moy |
| moy | 105,6 | 104,2 | 68,0 | 67,2 |
| Ecart type | 3,4 | 3,5 | 15,3 | 16,6 |
| Coef variation % | 3,2 | 3,4 | 22,5 | 24,6 |

**Une différence significative par rapport au D2 est constatée.**

| Ratio A/B | 50/50 - 0,5% Angle de contact (°) | | 50/50 - 25% Angle de contact (°) | |
|---|---|---|---|---|
| | reg | moy | reg | moy |
| moy | 77,6 | 76,8 | 69,5 | 68,0 |
| Ecart type | 13,2 | 12,2 | 7,1 | 6,5 |
| Coef variation % | 17,0 | 15,8 | 10,2 | 9,6 |

P =0.00355455**s P =0.0929457****ns

**Une différence significative par rapport au D2 est constatée pour le ratio 50/50 à 0.5%.**

On constate donc une différence significative du ratio 50/50 à 0,5% rincé par rapport au témoin décoloré, et une différence non significative par rapport au 50/50 1% Rincé.

RESULTATS DES TESTS HYDROPHOBICITE Tous les ratios rincés et non rincés à 1% +Essais 50/50 rincé à 0,5% et 0,25%

On constate un apport significatif d'hydrophobicité pour les traitements :

- Rincé à 1% : tous les ratios (moindre pour le 25/75)

- Rincé à 0.5% pour le ratio 50/50

- Non rincé à 1% tous les ratios (moindre pour les 75/25 et 0/100)

**Exemple 3 : Formulation d'un extrait d'Agave dans un sérum concentré pour soin végétal capillaire**

| Composants (nom INCI) | Pourcentage (% en poids) |
|---|---|
| AQUA | 55,575 |
| GELLAN GUM | 0,150 |
| SODIUM CHLORIDE | 0,075 |
| SODIUM BENZOATE | 0,500 |
| POTASSIUM SORBATE | 0,200 |
| SALICYLIC ACID | 0,150 |
| BETAINE | 2,000 |
| LACTIC ACID & AQUA | 0,150 |
| PANTHENOL | 0,200 |
| ALOE BARBADENSIS LEAF JUICE | 40,000 |
| INULIN | 1,000 |

**Exemple 4 : Formulation d'un extrait d'Agave dans un sérum concentré pour soin végétal capillaire**

| Composants (nom INCI) | Pourcentage (% en poids) |
|---|---|
| AQUA | 54,575 |
| GELLAN GUM | 0,150 |

(suite)

| Composants (nom INCI) | Pourcentage (% en poids) |
|---|---|
| SODIUM CHLORIDE | 0,075 |
| SODIUM BENZOATE | 0,500 |
| POTASSIUM SORBATE | 0,200 |
| SALICYLIC ACID | 0,150 |
| BETAINE | 2,000 |
| LACTIC ACID & AQUA | 0,150 |
| PANTHENOL | 0,200 |
| ALOE BARBADENSIS LEAF JUICE | 40,000 |
| FRUCTOOLIGOSACCHARIDES | 1,000 |
| INULIN | 1,000 |

**Exemple 5 : Formulation d'un extrait d'Agave dans un shampooing**

| Composants (nom INCI) | Pourcentage (% en poids) |
|---|---|
| AQUA | 76,660 |
| GUAR HYDROXYPROPYLTRIMONIUM CHLORIDE | 0,150 |
| CITRIC ACID | 0,090 |
| SODIUM BENZOATE | 0,500 |
| ZINC GLUCONATE | 0,500 |
| SALICYLIC ACID | 0,050 |
| FRUCTOOLIGOSACCHARIDES | 0,125 |
| INULIN | 0,125 |
| COCO-GLUCOSIDE & GLYCERYL OLEATE & AQUA | 3,000 |
| DECYL GLUCOSIDE & AQUA | 3,000 |
| AMMONIUM LAURYL SULFATE & AQUA | 10,400 |
| COCAMIDOPROPYL BETAINE & AQUA | 4,000 |
| PARFUM | 0,400 |
| URTICA DIOICA EXTRACT & AQUA & GLYCERIN | 1,000 |

**Exemple 6 : Formulation d'un extrait d'Agave dans un soin nutrition pour les cheveux sous forme d'après-shampooing**

| Composants (nom INCI) | Pourcentage (% en poids) |
|---|---|
| AQUA | 87,967 |
| INULIN | 0,250 |
| FRUCTOOLIGOSACCHARIDES | 0,250 |
| PANTHENOL | 0,100 |

EP 3 592 433 B1

(suite)

| Composants (nom INCI) | Pourcentage (% en poids) |
|---|---|
| SODIUM BENZOATE | 0,350 |
| BEHENTRIMONIUM CHLORIDE & ISOPROPYL ALCOHOL | 3,000 |
| STEARYL ALCOHOL | 3,500 |
| CETYL ALCOHOL | 2,000 |
| PERSEA GRATISSIMA OIL | 1,000 |
| COCOS NUCIFERA OIL | 1,000 |
| PARFUM | 0,400 |
| OLEA EUROPAEA FRUIT OIL & BETA-CAROTENE | 0,003 |
| CITRIC ACID | 0,180 |

**Listes des références**

**1.** WO200790554.

**2.** Maria de la Soledad ALONSO GUTIÉRREZ, thèse, « Valorisation de la bagasse de l'agave tequilana W. cv azul : caractérisation, étude de la digestibilité et de la fermentation des sucres », 2005.

**3.** López, M.G. ; Mancilla, N.A. ; Mendoza-Díaz, G. (2003). : Molecular structures of fructans from Agave tequilana Weber var. azul. J. Agric. Chem. 51, 7835-7840.

**4.** Prévention of hair surface aging, J. Cosmet. Sci., 62, 237-249 (March/April 2011), ERIK SCHULZE ZUR WIESCHE, ANDREA KÖRNER, KAROLA SCHÄFER, and FRANZ-JOSEF WORTMANN, Henkel AG & Co. KGaA, Hohenzollemring 127-129, 22763 Hamburg, Germany (E.S.z.W.), DWI an der RWTH Aachen e.V., Pauwelsstr. 8, 52056 Aachen, Germany (A.K., K.S.), and School of Materials, The University of Manchester, Manchester M13 9PL, UK (F-J.W.).

**Revendications**

**1.** Utilisation cosmétique non thérapeutique de fructanes *d'Agave tequilana* comprenant des inulines naturelles pour protéger la fibre capillaire par amélioration de l'hydrophobicité de la fibre capillaire.

**2.** Utilisation selon la revendication 1, dans laquelle lesdits fructanes ont un degré de polymérisation compris de 3 à 29.

**3.** Utilisation selon la revendication 1, dans laquelle lesdits fructanes sont des inulines ont un degré de polymérisation supérieur à 10.

**4.** Utilisation selon la revendication 3, dans laquelle lesdites inulines ont un degré de polymérisation compris de 10 à 60.

**5.** Utilisation selon la revendication 1, dans laquelle lesdits fructanes comprennent en outre des fructo-oligosaccharides ayant un degré de polymérisation inférieur ou égal à 10.

**6.** Utilisation selon l'une quelconque des revendications 3 à 5, dans laquelle le ratio inulines sur fructo-oligosaccharides est compris de 100:0 à 0:100, la borne 0:100 n'étant pas incluse.

**7.** Utilisation selon la revendication 6, dans laquelle le ratio inulines sur fructo-oligosaccharides est égal à 50:50.

**8.** Utilisation selon l'une quelconque des revendications précédentes, dans laquelle lesdits fructanes sont présents dans une composition cosmétique comprenant un support cosmétiquement acceptable.

**9.** Utilisation selon la revendication 8, dans laquelle ledit support cosmétiquement acceptable est choisi dans le groupe comprenant les shampooings, les onguents, les lotions, les crèmes, les huiles, les mousses, les laits, les pommades, les poudres, les solutions, les gels, les baumes, les beurres, les suspensions, les savons, les sprays, les émulsions et les sérums.

**10.** Procédé cosmétique non thérapeutique comprenant l'application thérapeutique de fructanes *d'Agave tequilana* comprenant des inulines ayant un degré de polymérisation supérieur à 10, pour protéger la fibre capillaire par amélioration de l'hydrophobicité de la fibre capillaire, ladite application étant éventuellement suivie d'un rinçage.

**11.** Procédé selon la revendication 10, dans lequel ladite application n'est pas suivie d'un rinçage.

**12.** Procédé selon la revendication 10 ou 11, sur des cheveux naturels, des cheveux abîmés, des cheveux secs, des cheveux colorés ou des cheveux cassants.

**Patentansprüche**

**1.** Nicht-therapeutische kosmetische Verwendung von Fruktanen aus Agave tequilana, die natürliche Inuline enthalten, zum Schutz der Haarfaser durch Verbesserung der Hydrophobie der Haarfaser.

**2.** Verwendung nach Anspruch 1, wobei die Fruktane einen Polymerisationsgrad von 3 bis 29 aufweisen.

**3.** Verwendung nach Anspruch 1, wobei die Fruktane Inuline mit einem Polymerisationsgrad von mehr als 10 sind.

**4.** Verwendung nach Anspruch 3, wobei die Inuline einen Polymerisationsgrad von 10 bis 60 aufweisen.

**5.** Verwendung nach Anspruch 1, wobei die Fructane ferner Fructooligosaccharide mit einem Polymerisationsgrad von weniger als oder gleich 10 umfassen.

**6.** Verwendung nach einem der Ansprüche 3 bis 5, wobei das Verhältnis von Inulinen zu Fructooligosacchariden zwischen 100:0 und 0:100 liegt, wobei der Grenzwert von 0:100 nicht eingeschlossen ist.

**7.** Verwendung nach Anspruch 6, wobei das Verhältnis von Inulinen zu Fructooligosacchariden gleich 50:50 ist.

**8.** Verwendung nach einem der vorhergehenden Ansprüche, **dadurch gekennzeichnet, dass** die Fructane in einer kosmetischen Zusammensetzung enthalten sind, die einen kosmetisch akzeptablen Träger enthält.

**9.** Verwendung nach Anspruch 8, **dadurch gekennzeichnet, dass** der kosmetisch akzeptable Träger ausgewählt ist aus der Gruppe, die Shampoos, Salben, Lotionen, Cremes, Öle, Mousses, Milchen, Salben, Pulver, Lösungen, Gele, Balsame, Butters, Suspensionen, Seifen, Sprays, Emulsionen und Seren umfasst.

**10.** Nicht-therapeutisches kosmetisches Verfahren, umfassend die therapeutische Anwendung von Fruktanen aus Agave tequilana, die Inuline mit einem Polymerisationsgrad von mehr als 10 umfassen, zum Schutz der Haarfaser durch Verbesserung der Hydrophobie der Haarfaser, wobei die Anwendung gegebenenfalls von einer Spülung gefolgt wird.

**11.** Verfahren nach Anspruch 10, wobei auf die Anwendung keine Spülung folgt.

**12.** Verfahren nach Anspruch 10 oder 11, bei natürlichem Haar, geschädigtem Haar, trockenem Haar, gefärbtem Haar oder brüchigem Haar.

**Claims**

**1.** The non-therapeutic cosmetic use of fructans of *Agave tequilana* comprising natural inulins for protecting the hair fiber by improving the hydrophobicity of the hair fiber.

**2.** The use as claimed in claim 1, wherein said fructans have a degree of polymerization of 3 to 29.

**3.** The use as claimed in claim 1, wherein said fructans are inulins having a degree of polymerization greater than 10.

4.  The use as claimed in claim 3, wherein said inulins have a degree of polymerization of 10 to 60.

5.  The use as claimed in claim 1, wherein said fructans further comprise fructooligosaccharides having a degree of polymerization less than or equal to 10.

6.  The use as claimed in any one of claims 3 to 5, wherein the ratio of inulins to fructooligosaccharides is from 100:0 to 0:100, the limit value of 0:100 not being included.

7.  The use as claimed in claim 6, wherein the ratio of inulins to fructooligosaccharides is equal to 50:50.

8.  The use as claimed in any one of the preceding claims, wherein said fructans are present in a cosmetic composition comprising a cosmetically acceptable support.

9.  The use as claimed in claim 8, wherein said cosmetically acceptable support is chosen from the group comprising shampoos, salves, lotions, creams, oils, mousses, milks, ointments, powders, solutions, gels, balms, butters, suspensions, soaps, sprays, emulsions and serums.

10. A non-therapeutic cosmetic process comprising the therapeutic application of fructans of *Agave tequilana* comprising inulins having a degree of polymerization greater than 10, for protecting the hair fiber by improving the hydrophobicity of the hair fiber, said application being optionally followed by rinsing.

11. The process as claimed in claim 10, wherein said application is not followed by rinsing.

12. The process as claimed in claim 10 or 11, on natural hair, damaged hair, dry hair, dyed hair or brittle hair.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 200790554 A

**Littérature non-brevet citée dans la description**

- **LÓPEZ, M.G. ; MANCILLA, N.A. ; MENDO-ZA-DÍAZ, G.** Molecular structures of fructans from Agave tequilana Weber var. azul. *J. Agric. Chem.,* 2003, vol. 51, 7835-7840
- Prévention of hair surface aging. **SCHULZE ZUR WIESCHE et al.** J. Cosmet. Sci. Henkel AG & Co. KGaA, Mars 2011, vol. 62, 237-249
- **MARIA DE LA SOLEDAD ALONSO GUTIÉRREZ.** Valorisation de la bagasse de l'agave tequilana W. cv azul : caractérisation, étude de la digestibilité et de la fermentation des sucres. *thèse,* 2005
- Prévention of hair surface aging. **ERIK SCHULZE ZUR WIESCHE ; ANDREA KÖRNER,KAROLA SCHÄFER ; FRANZ-JOSEF WORTMANN.** J. Cosmet. Sci. Henkel AG & Co. KGaA, Mars 2011, vol. 62, 237-249